(19) Europäisches Patentamt
European Patent Office
Office européen des brevets

(11) Veröffentlichungsnummer: **0 019 918**
**B1**

(12) # EUROPÄISCHE PATENTSCHRIFT

(45) Veröffentlichungstag der Patentschrift: **07.09.83**

(21) Anmeldenummer: **80103001.6**

(22) Anmeldetag: **29.05.80**

(51) Int. Cl.³: **C 07 D 241/44,** C 07 D 401/12, C 07 D 401/14, A 61 K 31/495 // (C07D401/12, 211/22, 241/44),(C07D401/14, 213/64, 211/22, 241/44, 235/26)

(54) **Neue Aminopropanol-Derivate, Verfahren zu ihrer Herstellung und diese Verbindungen enthaltende Arzneimittel.**

(30) Priorität: **31.05.79 DE 2922084**

(43) Veröffentlichungstag der Anmeldung: **10.12.80 Patentblatt 80/25**

(45) Bekanntmachung des Hinweises auf die Patenterteilung: **07.09.83 Patentblatt 83/36**

(84) Benannte Vertragsstaaten: **AT BE CH DE FR GB IT LI LU NL SE**

(56) Entgegenhaltungen:
**AT-B-242 150**
**DE-A-1 670 919**
**US-A-3 366 628**
**US-A-3 635 971**
**US-A-4 146 630**

**Die Akte enthält technische Angaben, die nach dem Eingang der Anmeldung eingereicht wurden und die nicht in dieser Patentschrift enthalten sind.**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof (DE)**

(72) Erfinder: **Ross, Carl Heinz, Dr. rer nat., Rathausstrasse 45, D-6806 Viernheim (DE)**
Erfinder: **Kampe, Wolfgang, Dr. rer. nat., Zedernstrasse 49, D-6805 Heddesheim (DE)**
Erfinder: **Bartsch, Wolfgang, Dr. med. vet., Franconviller-Strasse 5, D-6806 Viernheim (DE)**
Erfinder: **Sponer, Gisbert, Dr. med. vet., Tilsiterstrasse 30, D-6944 Hemsbach (DE)**
Erfinder: **Roesch, Egon, Dr. med., Am Oberen Luisenpark 22, D-6800 Mannheim 1 (DE)**

EP 0 019 918 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neue Aminopropanol-Derivate, Verfahren zu ihrer Herstellung und diese Verbindungen enthaltende Arzneimittel

Die vorliegende Erfindung betrifft neue Aminopropanol-Derivate der allgemeinen Formel (I)

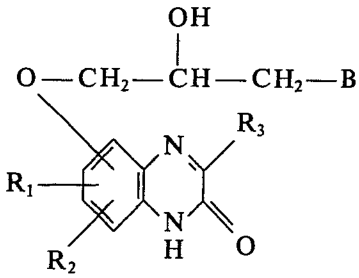

(I)

in der

| | |
|---|---|
| $R_1$ und $R_2$ | die gleich oder verschieden sein können, jeweils ein Wasserstoffatom oder eine $C_1-C_5$-Alkylgruppe oder gemeinsam einen $C_2-C_4$-Alkylenrest, |
| $R_3$ | Wasserstoff oder einen $C_1-C_5$-Alkylrest, der gegebenenfalls durch Hydroxyl, Halogen, Phenyl oder $C_1-C_5$-Alkylthio substituiert ist, und |
| B | einen $C_2-C_4$-Alkylaminorest, dessen Alkylteil gegebenenfalls einen Phenyl- oder Phenoxyrest trägt, der ein- oder mehrfach durch Halogen, Hydroxyl, $C_1-C_4$-Alkyl, $C_2-C_6$-Alkanoyl, Methylthio, $C_2-C_6$-Alkanoylamido, Aminocarbonyl, $C_1-C_4$-Alkoxy, Allyloxy, Phenoxy oder Trifluormethyl substituiert ist, oder einen Phenyloxymethylpiperidin-, Pyridyloxymethylpiperidin- oder Benzimidazolinonyloxymethylpiperidinrest, wobei der Phenylrest gegebenenfalls ein- oder mehrfach durch Halogen, Amino, Hydroxy, Carboxamido, eine $C_1-C_4$-Alkyl-, $C_2-C_6$-Alkanoyl- oder $C_1-C_4$-Alkoxygruppe substituiert ist, |

bedeuten, deren pharmakologisch unbedenkliche Salze, Verfahren zur Herstellung derselben sowie pharmazeutische Zubereitungen, die Substanzen der Formel (I) enthalten.

Verbindungen der Formel (I) enthalten ein asymmetrisches Kohlenstoffatom und können daher in optisch aktiver Form oder als racemisches Gemisch vorliegen. Gegenstand der vorliegenden Anmeldung sind sowohl die racemischen Formen als auch die optischen Isomeren.

Von den Alkylaminoresten, die B definieren, sind Isopropylamin, tert.-Butylamin, sek.-Butylamin, und soweit diese gemäß Anspruch 1 substituierte und unsubstituierte Phenyl- und Phenoxyalkylaminreste betreffen sind Phenyl- und Phenoxyethylamin- und -propylaminreste bevorzugt.

Die Alkyl- bzw. Alkoxygruppen, die in den Definitionen der Substituenten $R_1$, $R_2$, $R_3$ und B auftreten, können geradkettig oder verzweigt sein; $R_3$ enthält vorzugsweise 1–4 Kohlenstoffatome. Bevorzugt ist die Methyl- bzw. Methoxy-, Ethoxy- und Propoxygruppe.

Der durch die Substituenten $R_1$ und $R_2$ gegebenenfalls gebildete Alkylenrest mit 2–4 Kohlenstoffatomen betrifft vorzugsweise die Ortho-Verknüpfung.

Unter Halogen wird im Sinne der Erfindung Fluor, Chlor, Brom und Jod verstanden, insbesondere Fluor, Chlor und Brom.

Die Verbindungen der allgemeinen Formel (I) sowie ihre pharmakologisch unbedenklichen Salze hemmen adrenergische $\beta$-Rezeptoren und senken gleichzeitig in hohem Maße den Blutdruck. Sie eignen sich daher zur Behandlung oder Prophylaxe bei Herz- und Kreislauferkrankungen.

Es ist bekannt, daß Aminopropanole ähnlicher Struktur ähnliche Wirkungen haben. So sind z. B. aus US-A-4 146 630 entsprechende Indole, Indazole, Benzimidazole und Benzotriazole bekannt. Durch Einführung der neuen heterocyclischen Phenolkomponenten wurde jedoch eine überraschende Verbesserung der Wirkqualität erreicht.

Die Herstellung der neuen Verbindungen der allgemeinen Formel I ist dadurch gekennzeichnet, daß man in an sich bekannter Weise entweder

a) eine Verbindung der allgemeinen Formel (II)

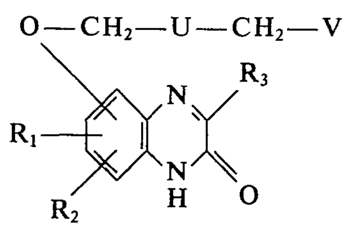

(II)

mit einer Verbindung der allgemeinen Formel (III)

$$H-B \qquad \text{(III)}$$

umsetzt, in denen

$R_1$, $R_2$, $R_3$ und B die oben angegebene Bedeutung haben,
V einen nucleophil substituierbaren Rest und
U die Gruppe $>C=O$ oder $>CH-OH$ darstellt oder
U zusammen mit V eine Einfachbindung bildet, und falls U die Gruppe $>C=O$ bedeutet, anschließend reduziert, oder

b) eine Verbindung der allgemeinen Formel (IV)

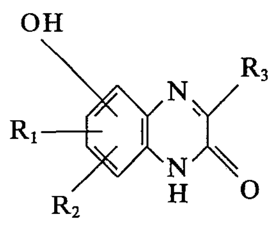

(IV)

mit einer Verbindung der allgemeinen Formel (V)

$$V-CH_2-U-CH_2-B \qquad \text{(V)}$$

umsetzt, in denen

$R_1$, $R_2$, $R_3$, B,
U und V die oben angegebene Bedeutung haben, und falls U die Gruppe $>C=O$ bedeutet, anschließend reduziert, oder

c) für den Fall, daß $R_3=CH_3$ vorstellt, eine Verbindung der allgemeinen Formel (VI)

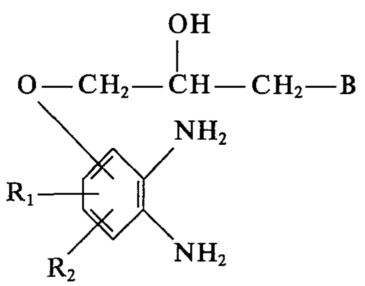

(VI)

in der

$R_1$, $R_2$ und B die angegebene Bedeutung haben, mit Acetylendicarbonsäure umsetzt, oder

d) eine Verbindung der allgemeinen Formel (VI) mit einer Verbindung der allgemeinen Formel (VII)

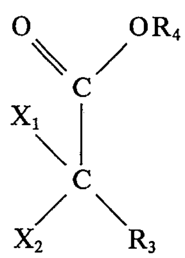

(VII)

in der

$R_3$ die oben angegebene Bedeutung hat,
$R_4$ Wasserstoff oder $C_1-C_4$-Alkyl,
$X_1$ Halogen und
$X_2$ Wasserstoff oder $X_1$ und $X_2$ zusammen ein Sauerstoffatom bedeuten,

umsetzt, und falls $X_2$ Wasserstoff darstellt, oxidiert, oder

e) eine Verbindung der allgemeinen Formel (VIII)

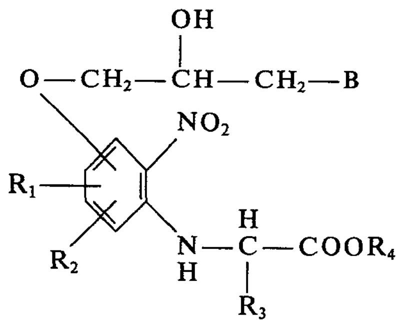

(VIII)

in der

$R_1$, $R_2$, $R_3$, $R_4$ und B die oben angegebene Bedeutung haben, reduziert, cyclisiert und oxidiert, oder

f) eine Verbindung der allgemeinen Formel (IX)

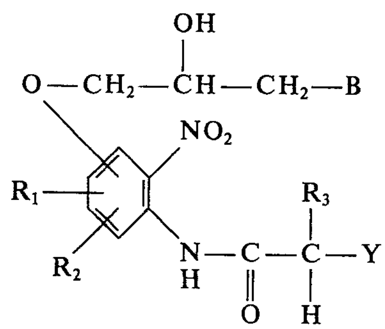

(IX)

in der

$R_1$, $R_2$, $R_3$ und B die oben angegebene Bedeutung haben und
Y für eine nucleophil substituierbare Gruppe steht, reduziert, cyclisiert und oxidiert,

und anschließend einen Substituenten im Rest B gewünschtenfalls in einen anderen Substituenten umwandelt und gegebenenfalls die erhaltenen Verbindungen der allgemeinen Formel (I) in ihre pharmakologisch verträglichen Salze überführt.

V und Y in Verbindungen der allgemeinen Formeln (II), (V) und (IX) stehen für alle Reste, die sich nucleophil substituieren lassen. Solche Reste sind z. B. Halogenatome, vorzugsweise Brom oder Chlor, oder Sulfonsäureestergruppen.

Die erfindungsgemäßen Verfahren werden zweckmäßig in einem unter den Reaktionsbedingungen inerten Lösungsmittel, z. B. Wasser, Ethanol, Dioxan oder Dimethylformamid, gegebenenfalls in Gegenwart eines säurebindenden Mittels durchgeführt. Die Umsetzungen können auch nach Mischen der Reaktionskomponenten ohne Lösungsmittel erreicht werden. Die Reaktionen werden durch Stehenlassen bei Raumtemperatur oder durch Erwärmen, gegebenenfalls unter einer Schutzgasatmosphäre ausgeführt.

Die Umsetzung der Verbindungen der Formel (IV) mit den Substanzen der Formel (V) gemäß Verfahren b) erfolgt zweckmäßig unter Sauerstoffausschluß in Gegenwart eines Säureakzeptors. Man kann aber auch Alkalisalze der Hydroxyverbindungen der Formel (IV) einsetzen.

Die gegebenenfalls durchzuführende Reduktion der Gruppe $>C=O$ erfolgt durch katalytische Hydrierung mit Edelmetall- oder Nickelkatalysatoren oder mittels komplexer Metallhydride, wie z. B. Natriumborhydrid. Reduktionen wie sie für Verfahren e) und f) notwendig sind, werden vorzugsweise mit katalytisch erregtem Wasserstoff durchgeführt.

Das Verfahren e) wird vorzugsweise unter den Bedingungen der Hydrierung durchgeführt; die Cyclisierung erfolgt gegebenenfalls unter Säurezusatz.

Die Cyclisierung gemäß Verfahren f) geschieht unter Zusatz von Basen wie z. B. Triethylamin oder Kaliumcarbonat.

Oxidationen, die für Verfahren d), e) und f) von Bedeutung sind, werden zweckmäßig mit Luft, Wasserstoffperoxid im Alkalischen oder Kaliumpermanganat durchgeführt.

Verbindungen der allgemeinen Formel (VI) sind zum Teil bekannte Verbindungen (vgl.

…E-A-2 737 630.3) oder können analog den dort beschriebenen Verfahren hergestellt werden.

Verbindungen der allgemeinen Formel (IV) sind bekannt oder können analog Verfahren c), d), e) oder f) aus bekannten Phenolen hergestellt werden.

Verbindungen der allgemeinen Formel (IX) sind neu und können aus den entsprechenden 2-Nitro-anilinen durch Umsetzung mit 2-Halogencarbonsäurechloriden erhalten werden.

Als nachträgliche Umwandlung eines Substituenten in dem Rest B sei z. B. die Überführung einer Aminogruppe in eine Alkylcarbonylaminogruppe erwähnt. Diese Umsetzungen erfolgen ebenfalls nach bekannten Methoden mit üblichen Acylierungsmitteln, wie z. B. Carbonsäureanhydriden, Carbonsäurechloriden.

Als gegebenenfalls notwendige Schutzgruppen können im Prinzip alle zum intermediären Schutz von Amino- bzw. Hydroxygruppen verwendeten Schutzgruppen eingesetzt werden, die leicht wieder abspaltbar sind. Bevorzugt ist die Benzylgruppe, die sich nach der Reaktion gemäß einem der angegebenen Verfahren in an sich bekannter Weise hydrogenolytisch abspalten läßt.

Die erfindungsgemäßen Verbindungen der Formel (I) können in Form eines racemischen Gemisches anfallen. Die Trennung des Racemats in die optisch aktiven Formen geschieht nach an sich bekannten Methoden über die diastereomeren Salze aktiver Säuren, wie z. B. Weinsäure, Apfelsäure oder Camphersulfonsäure.

Die neuen Verbindungen der allgemeinen Formel (I) fallen unter den Reaktionsbedingungen der beschriebenen Verfahren vorwiegend als Säureadditionssalze an, z. B. als Hydrochloride, und können nach beschriebenen Methoden ohne weiteres in die freien Basen überführt werden.

Zur Überführung der Verbindungen der allgemeinen Formel (I) in ihre pharmakologisch unbedenklichen Salze setzt man diese, vorzugsweise in einem organischen Lösungsmittel, mit der äquivalenten Menge einer anorganischen oder organischen Säure, z. B. Salzsäure, Bromwasserstoffsäure, Phosphorsäure, Schwefelsäure, Essigsäure, Citronensäure, Maleinsäure um.

Zur Herstellung von Arzneimitteln werden die Substanzen (I) in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z. B. Olivenöl, suspendiert oder gelöst.

Die erfindungsgemäßen neuen Substanzen (I) und ihre Salze können in flüssiger oder fester Form enteral oder parenteral appliziert werden. Als Injektionsmedium kommt vorzugsweise Wasser zur Anwendung, welches die bei Injektionslösungen üblichen Zusätze wie Stabilisierungsmittel, Lösungsvermittler oder Puffer enthält.

Derartige Zusätze sind z. B. Tartrat- und Citratpuffer, Ethanol, Komplexbildner (wie Ethylendiamintetraessigsäure und deren nichttoxische Salze), hochmolekulare Polymere (wie flüssiges Polyethylenoxyd) zur Viskositätsregulierung. Feste Trägerstoffe sind z. B. Stärke, Lactose, Mannit, Methylcellulose, Talkum, hochdisperse Kieselsäuren, hochmolekulare Fettsäuren (wie Stearinsäure), Gelatine, Agar-Agar, Calciumphosphat, Magnesiumstearat, tierische und pflanzliche Fette und feste hochmolekulare Polymere (wie Polyethylenglykole). Für orale Applikation geeignete Zubereitungen können gewünschtenfalls Geschmacks- und Süßstoffe enthalten.

Die Dosierung kann von verschiedenen Faktoren, wie Applikationsweise, Spezies, Alter und/oder individuellem Zustand abhängen. Die täglich zu verabreichenden Dosen liegen bei oraler Applikation zwischen etwa 5 mg und etwa 50 mg für Warmblüter mit einem Gewicht von etwa 70 kg.

Die vasodilatierende und $\beta$-rezeptoren-blockierende Wirksamkeit der Verbindungen der allgemeinen Formel (I) gemäß der vorliegenden Erfindung wurde durch die folgenden 2 Versuche gezeigt, da beide Eigenschaften nicht an einem Modell demonstriert werden können:

### 1. Prüfung auf $\beta$-blockierende Qualität

Kaninchen wurden in Holzkäfigen fixiert, die Herzfrequenz wurde über Stichelektroden abgeleitet und auf einem Frequenzzählgerät (Meßzeit 15 sec) abgelesen. Über eine Ohrvene wurde zunächst 1 µg/kg i. v. Isoprenalin injiziert, was eine Erhöhung der Herzfrequenz von ca. 210 Schläge/min auf ca. 350 Schläge/min bewirkt. Anschließend wurden die Prüfsubstanzen in steigender Dosierung (1+1+2+4+8 ... Einheiten — die erste Dosis war meist 125 µg/kg i. v., bei hochwirksamen Substanzen aber auch entsprechend geringer) intravenös verabreicht und die Herzfrequenz nach Isoprenalin-Gabe erneut abgelesen. Die Hemmung der Isoprenalin-Tachycardie ist als Maß für die $\beta$-Blockade anzusehen. Es wurde die Dosis der Prüfsubstanzen bestimmt, die den Anstieg der Herzfrequenz unter Isoprenalin (1 µg/kg i. v.) um 30 bzw. 50% abschwächt ($HD_{30 \text{ bzw. } 50\%}$).

### 2. Prüfung auf vasodilatierende Aktivität

Kaninchen wurden mit Urethan narkotisiert. Zur fortlaufenden Messung des arteriellen Blutdruckes wurde ein Katheter in die A. femoralis implantiert. Die Messung des Blutdruckes erfolgte mittels eines elektromechanischen Druckwandlers (Statham P 23 Db). Die Impulse wurden auf einem Direktschreiber aufgezeichnet und nach Eichen mit einem Quecksilbermanometer ausgewertet.

Nach Ermittlung des Ausgangswertes wurden beide Halsschlagadern (A. carotides) für 2 Minuten occludiert und auf diese Weise der Blutdruck temporär erhöht (CSE-Reflex). Anschließend wurde die Prüfsubstanz in der niedrigsten Prüfdosis (siehe unter 1.) i. v. injiziert und weiter 8 Minuten später der CSE-Reflex erneut ausgelöst.

Substanzen, die unter diesen Bedingungen den Anstieg des Blutdruckes unter CSE abschwächen, können als vasodilatierend angesehen werden. Errechnet wurde von den Prüfsubstanzen die Dosis, die den CSE-Reflex um 30 mm Hg abschwächt ($ED_{-30\ mm\ Hg}$).

Um einen Vergleich gegenüber dem Stand der Technik zu ermöglichen, wurden die Ergebnisse aus dem 1. Versuch mit denen eines gut wirksamen Handelsprodukts in Relation gesetzt. (Hierzu siehe RP = relative Potenz zu Propranolol (1-Isopropylamino-3-(1-naphthyloxy)-2-propanol), d. h. Wirkdosis Propranolol $HD_{50\ fcor}$ 393 µg/kg i. v. = 1.)

Die Ergebnisse sind der folgenden Tabelle zu entnehmen.

| Beispiel | $HD_{30\ f_{cor}}$ µg/kg i.v. | $HD_{50\ f_{cor}}$ µg/kg i.v. | RP | $ED_{-30}$ µg/kg i.v. |
|---|---|---|---|---|
| Propranolol | 92 | 393 | 1 | >3 550 |
| 1.1. Verb. | 3,0 | 12,3 | 30 | – |
| 1.2. Verb. | 4,4 | 18,4 | 22 | – |
| 1 a.1. Verb. | 156 | 588 | 0,66 | 14 900 |
| 1 b.1. Verb. | 400 | 1 091 | 0,36 | 7 900 |
| 1 c.1. Verb. | 52 | 212 | 1,85 | 1 240 |
| 1 e.1. Verb. | 152 | 472 | 0,8 | – |
| 1 f.1. Verb. | 38 | 180 | 2,2 | 1 940 |
| 1 g.1. Verb. | 173 | 669 | 0,6 | 513 |

Bevorzugt im Sinne der vorliegenden Anmeldung sind außer den in den Beispielen genannten Verbindungen die folgenden:

5-⟨2-Hydroxy-3-[4-(2-methoxyphenoxymethyl)piperidino]propoxy⟩-3-methyl-2-chinoxalinon
5-⟨2-Hydroxy-3-[4-(2-methoxy-4-methyl-phenoxymethyl)piperidino]propoxy⟩-3-methyl-2-chinoxalinon
5-⟨2-Hydroxy-3-[2-(2-methoxyphenoxy)ethylamino]propoxy⟩-2-chinoxalinon
5-⟨2-Hydroxy-3-[2-(2-methoxyphenoxy)ethylamino]propoxy⟩-3-hydroxymethyl-2-chinoxalinon
5-⟨2-Hydroxy-3-[2-(2-methoxyphenoxy)propylamino]propoxy⟩-3-methyl-2-chinoxalinon
5-⟨2-Hydroxy-3-[2-(2-hydroxyphenoxy)propylamino]propoxy⟩-3-methyl-2-chinoxalinon
5-[2-Hydroxy-3-(2-phenoxyethylamino)propoxy]-3-methyl-2-chinoxalinon
5-⟨2-Hydroxy-3-[2-(4-hydroxy-2-methyl-phenoxy)ethylamino]propoxy⟩-3-methyl-2-chinoxalinon
5-⟨2-Hydroxy-3-[2-(2-allyloxy-phenoxy)ethylamino]propoxy⟩-3-methyl-2-chinoxalinon
5-⟨2-Hydroxy-3-[2-(2-isopropoxy-phenoxy)ethylamino]propoxy⟩-3-methyl-2-chionoxalinon
5-⟨2-Hydroxy-3-[2-(2-phenoxy-phenoxy)ethylamino]propoxy⟩-3-methyl-2-chinoxalinon
5-⟨2-Hydroxy-3-[2-(2,6-dimethoxy-phenoxy)ethylamino]propoxy⟩-3-methyl-2-chinoxalinon
5-⟨2-Hydroxy-3-[2-(2-trifluormethyl-phenoxy)ethylamino]propoxy⟩-3-methyl-2-chinoxalinon
5-⟨2-Hydroxy-3-[2-(2-acetamido-phenoxy)ethylamino]propoxy⟩-3-methyl-2-chinoxalinon
5-⟨2-Hydroxy-3-[2-(2-methylthio-phenoxy)ethylamino]propoxy⟩-3-methyl-2-chinoxalinon

Die Erfindung wird durch die folgenden Beispiele näher erläutert. Sie zeigen einige der zahlreich möglichen Verfahrensvarianten, die zur Synthese der erfindungsgemäßen Verbindungen verwendet werden können.

Beispiel 1

1) 5-(2-Hydroxy-3-tert-butylamino-propoxy)-3-methyl-2-chinoxalinon-hydrochlorid

und

2) 8-(2-Hydroxy-3-tert-butylamino-propoxy)-3-methyl-2-chinoxalinon-hydrochlorid

7,2 g 2,3-Diamino-1-(2-hydroxy-3-tert-butylamino-propoxy)-benzol-trihydrochlorid werden in 40 ml Wasser in der Hitze gelöst. Zur heißen Lösung werden 2,28 g Acetylendicarbonsäure, gelöst in 20 ml Wasser, gegeben. Nach 20 Stunden wird i. Vak. zur Trockene eingeengt und aus Ethanol fraktioniert kristallisiert. Ein erstes Kristallisat von 3,1 g schmilzt nach Umkristallisation aus Ethanol unter Zusatz von Aktivkohle bei 274–275° C. Diese Verbindung ist das 5-(2-Hydroxy-3-tert-butylamino-propoxy)-3-methyl-2-chinoxalinon-hydrochlorid.

Ein Zweitkristallisat (0,83 g) mit Schmelzpunkt 225–228° C entspricht dem 8-(2-Hydroxy-3-tert-butylamino-propoxy)-3-methyl-2-chinoxalinon-hydrochlorid.

In analoger Weise werden aus substituierten o-Phenylendiaminen mit Acetylendicarbonsäure erhalten:

| | Bezeichnung | Schmelzpunkt ° C (Lösungsmittel) |
|---|---|---|
| a 1) | 5-‹2-Hydroxy-3-[2-(3,4-dimethoxyphen)-ethylamino]propoxy›-3-methyl-2-chinoxalinon<br>und | 163–164<br>(Ethanol) |
| a 2) | 8-‹2-Hydroxy-3-[2-(3,4-dimethoxyphen)-ethylamino]propoxy›-3-methyl-2-chinoxylinon-hydrochlorid<br>aus<br>2,3-Diamino-1-‹2-hydroxy-3-[2-(3,4-dimethoxyphen)-ethylamino]-propoxy›-benzol-trihydrochlorid | 229–231<br>(Ethanol) |
| b 1) | 5-‹2-Hydroxy-3-[2-(3,4-dimethoxyphen)-ethylamino]propoxy›-3,8-dimethyl-2-chinoxylinon-hydrochlorid<br>und | 218–220<br>(Ethanol/ Methanol) |
| b 2) | 8-‹2-Hydroxy-3-[2-(3,4-dimethoxyphen)-ethylamino]propoxy›-3,5-dimethyl-2-chinoxylinon-hydrochlorid<br>(Reinigung über Kieselgelsäule mit Chloroform/Methanol 8 : 2 als Laufmittel)<br>aus<br>2,3-Diamino-‹2-Hydroxy-3-[2-(3,4-dimethoxyphen)-ethylamino]-propoxy›-4-methyl-benzoltrihydrochlorid | 228–230<br>(Ethanol/ Isopropanol) |
| c 1) | 5-‹2-Hydroxy-3-[2-(2-hydroxyphenoxy)ethylamino]propoxy›-3,8-dimethyl-2-chinoxylinon-hydrochlorid<br>und | 263–265<br>(Ethanol/ Methanol) |
| c 2) | 8-‹2-Hydroxy-3-[2-(2-hydroxyphenoxy)ethylamino]propoxy›-3,5-dimethyl-2-chinoxylinon-hydrochlorid<br>aus<br>2,3-Diamino-‹2-Hydroxy-3-[2-(2-hydroxyphenoxy)ethylamino]-propoxy›-4-methyl-benzol-trihydrochlorid | 272–273<br>(Ethanol/ Methanol) |
| d 1) | 5-‹2-Hydroxy-3-[4-(2-pyridyloxymethyl)piperidino]propoxy›-3-methyl-2-chinoxalinon<br>und | 194–119<br>(Ethanol) |
| d 2) | 8-‹2-Hydroxy-3-[4-(2-pyridyloxymethyl)piperidino]propoxy›-3-methyl-2-chinoxylinon<br>Reinigung über Kieselgelsäule mit Chloroform/Methanol 8 : 2 als Laufmittel)<br>aus<br>2,3-Diamino-1-‹2-hydroxy-3-[4-(2-pyridyloxymethyl)piperidino]-propoxy›-benzol-trihydrochlorid | 190–192<br>(Methanol) |

Fortsetzung

| | Bezeichnung | Schmelzpunkt ° C (Lösungsmittel) |
|---|---|---|
| e 1) | 5-‹2-Hydroxy-3-[4-(phenoxymethyl)piperidino]propoxy›-3-methyl-2-chinoxylinon | 174–177 (Methanol) |
| | und | |
| e 2) | 8-‹2-Hydroxy-3-[4-(phenoxymethyl)piperidino]propoxy›-3-methyl-2-chinoxalinon | 207–209 (Methanol) |
| | aus 2,3-Diamino-1-‹2-Hydroxy-3-[4-phenoxymehtyl)piperidino]propoxy›-benzol-trihydrochlorid | |
| f 1) | 5-‹2-Hydroxy-3-[2-(2-hydroxyphenoxy)ethylamino]propoxy›-3-methyl-2-chinoxalinon | 193–194 (Ethanol) |
| | und | |
| f 2) | 8-‹2-Hydroxy-3-[2-(2-hydroxyphenoxy)ethylamino]propoxy›-3-methyl-2-chinoxalinon-hydrochlorid | 238–240 (Methanol) |
| | aus 2,3-Diamino-‹2-Hydroxy-3-[2-(2-hydroxyphenoxy)ethylamino]-propoxy›-benzol-trihydrochlorid | |
| g 1) | 5-‹2-Hydroxy-3-[2(2-methoxyphenoxy)ethylamino]propoxy›-3-methyl-2-chinoxalinon-hydrochlorid | 197–199 (Methanol) |
| | und | |
| g 2) | 8-‹2-Hydroxy-3-[2(2-methoxyphenoxy)ethylamino]propoxy›-3-methyl-2-chinoxalinon-hydrochlorid | 253–254 (Methanol) |
| | aus 2,3-Diamino-1-‹2-Hydroxy-3-[2(2-methoxyphenoxy)ethylamino]-propoxy›-benzol-trihydrochlorid | |
| h 1) | 5-(2-Hydroxy-3-(S)-2-butylaminopropoxy)-3-methyl-2-chinoxalinon-hydrochlorid | 256–258 (Ethanol/Ether) |
| | und | |
| h 2) | 8-(2-Hydroxy-3-(S)-2-butylaminopropoxy)-3-methyl-2-chinoxalinon-hydrochlorid | 190–193 (Etanol/Ether) |
| | aus 2,3-Diamino-1-(2-hydroxy-3-(S)-2-butylaminopropoxy)benzol-trihydrochlorid | |
| i 1) | 5-(2-Hydroxy-3-(R)-2-butylaminopropoxy)-3-methyl-2-chinoxalinon-hydrochlorid | 255–257 (Ethanol/Ether) |
| | und | |
| i 2) | 8-(2-Hydroxy-3-(R)-2-butylaminopropoxy)-3-methyl-2-chinoxalinon-hydrochlorid | 191–194 (Ethanol/Ether) |
| | aus 2,3-Diamino-1-(2-Hydroxy-3-(R)-2-butylaminopropoxy)benzol-trihydrochlorid (Trennung über Kieselgelsäule mit Chloroform/Methanol 8 : 2 als Laufmittel) | |
| k 1) | 5-‹2-Hydroxy-3-[4-(4-(2)-benzimidazolinoyl-oxymethyl)piperidino]-propoxy›-3-methyl-2-chinoxalinon | |
| | und | |
| k 2) | 8-‹2-Hydroxy-3-[4-(4-(2)-benzimidazolinoyl-oxymethyl)piperidino]-propoxy›-3-methyl-2-chinoxalinon | 253–254 (Methanol) |
| | aus 2,3-Diamino-1-‹2-Hydroxy-3-[4-(4-(2)-benzimidazolinoyl-oxymethyl)-piperidino]propoxy›-benzol-trihydrochlorid | |
| l 1) | 5-[2-Hydroxy-3-(2-phenoxyethylamino)propoxy]-3,7-dimethyl-2-chinoxalinon-hydrochlorid | 226–229 (Ethanol/ Methanol) |
| | und | |

Fortsetzung

| | Bezeichnung | Schmelzpunkt °C (Lösungsmittel) |
|---|---|---|
| l 2) | 8-[2-Hydroxy-3-(2-phenoxyethylamino)propoxy]-3,6-dimethyl-2-chinoxalinon-hydrochlorid aus 2,3-Diamino-2-[2-Hydroxy-3-(2-phenoxyethylamino)propoxy]-5-methyl-benzol-trihydrochlorid | |

Beispiel 2

5-⟨2-Hydroxy-3-[2-(3,4-dimethoxyphen)ethylamino]propoxy⟩-3,8-dimethyl-2-chinoxalinon-hydrochlorid

28,0 g 2,3-Dinitro-1-⟨2-hydroxy-3-[2-(N-benzyl-3,4-dimethoxyphen)ethylamino]propoxy⟩-4-methylbenzol wird mit 15,9 g Alanin, 15,0 g Natriumhydrogencarbonat und 250 ml Ethylenglykolmonomethylether 4 Stunden am Rückflußkühler gekocht. Das Reaktionsgemisch wird in 600 ml Wasser eingegossen. Den Chloroformextrakt (6 × 100 ml) trocknet man über Natriumsulfat. Nach vollständigem Einengen im Vakuum erhält man 32,0 g öligen Rückstand, der in 280 ml Ethanol aufgenommen und mit 4 ml konz. Schwefelsäure versetzt wird. Nach 4stündigem Rückflußkochen wird zur Trockene abdestilliert, in 300 ml Chloroform aufgenommen, mit Wasser, 10proz. Natriumhydrogencarbonat-Lösung und wieder Wasser gewaschen und nach Trocknen über Natriumsulfat und Klären mit Aktivkohle eingeengt. Man erhält 33,5 g öligen 2-⟨⟨6-⟨2-Hydroxy-3-[2-(N-benzyl-3,4-dimethoxyphen)ethylamino]-propoxy⟩-3-methyl-2-nitro-anilino⟩⟩-propionsäureethylester.

33,0 g 2-⟨⟨6-⟨2-Hydroxy-3-[2-(N-benzyl-3,4-dimethoxyphen)ethylamino]propoxy⟩-3-methyl-2-nitro-anilino⟩⟩-propionsäureethylester werden in 400 ml Ethanol über 3,5 g Platindioxid und anschließend über 8,0 g 10proz. Palladiumkohle bei Normaldruck und 50°C hydriert. Man befreit vom Katalysator, säuert mit 2 N Salzsäure an und rührt heftig 1 Stunde unter Zusatz von Aktivkohle. Man filtriert und engt auf 200 ml ein. Die ausgefallenen Kristalle (7,0 g entsprechend 25% d. Th. bezogen auf eingesetzte Dinitroverbindung) sind identisch (Dünnschichtchromatogramm und Mischschmelzpunkt) mit der in Beispiel 1 b 1 erhaltenen Verbindung.

Beispiel 3

5-⟨2-Hydroxy-3-[2-(2-methoxyphenoxy)ethylamino]propoxy⟩-3-methyl-2-chinoxalinon-hydrochlorid

52,3 g 2,3-Dinitro-1-⟨2-hydroxy-3-[N-benzyl-2-(2-methoxyphenoxy)ethylamino]propoxy⟩-benzol, 26,7 g Alanin, 25,2 g Natriumhydrogencarbonat, 400 ml Ethanol und 200 ml Wasser werden 5 Stunden unter Rückfluß gekocht. Es wird vollständig eingeengt, in 800 ml Wasser und 300 ml Ethylenglykolmonomethylether gelöst und mit insgesamt 1500 ml Dichlormethan ausgezogen. Aus der org. Phase erhält man nach Trocknen über Natriumsulfat und Einengen 56,2 g (100%) Natriumsalz der 2-{6-⟨2-Hydroxy-3-[N-benzyl-2-(2-methoxyphenoxy)ethylamino]propoxy⟩2-nitroanilino}-propionsäure.

31,2 g dieses Salzes in 450 ml Ethanol und 50 ml Wasser werden bei 50°C und 5 bar über 6 g Raneynickel hydriert. Nach Filtration und Einstellen auf pH 4 mit 2 N Salzsäure wird über 8 g 5proz. Palladiumkohle bei 60°C und Normaldruck die Schutzgruppe abhydriert. Man engt zur Trockene ein, nimmt in 250 ml Ethanol auf, befreit vom abgeschiedenen Salz, leitet Luft durch die Lösung und saugt nach 10stündigem Stehen die ausgefallenen Kristalle ab. Man erhält 5,65 g (23%), die nach nochmaligem Umkristallisieren aus Ethanol mit der Verbindung aus Beispiel 1 g 1 identisch sind.

Beispiel 4

5-⟨2-Hydroxy-[2-(2-methoxyphenoxy)ethylamino]propoxy⟩-3-(2)-propyl-2-chinoxalinon-hydrochlorid

Analog Beispiel 3 wird aus 1-⟨2-Hydroxy-[N-benzyl-2-(2-methoxyphenoxy)ethylamino]propoxy⟩-2,3-dinitrobenzol und Valin die Titelverbindung vom Schmelzpunkt 174—176°C (Ethanol) erhalten.

Beispiel 5

| Wirkstoffhaltige Tabletten | | für 1 Tabl. | für 100 000 Tabl. |
|---|---|---|---|
| I | Wirkstoff [5-‹2-Hydroxy-3-[2-(2-methoxyphenoxy)-ethylamino]propoxy›-3-methyl-2-chinoxalinon-hydroclorid] | 10,000 mg | 1,000 kg |
| | Lactose | 67,000 mg | 6,700 kg |
| | Maisstärke | 35,000 mg | 3,500 kg |
| II | Polyvinylpyrrolidon, Mol-Gew. 30 000 | 3,000 mg | 0,300 kg |
| III | Natriumcarboxymethylamylopektin | 4,000 mg | 0,400 kg |
| | Cellulosepulver | 20,000 mg | 2,000 kg |
| | Magnesiumstearat | 1,000 mg | 0,100 kg |
| | | 140,000 mg | 14,000 kg |
| | Wasser zum Granulieren | | 1,000 kg |

Herstellung: Die Substanzen I werden mit der wäßrigen Lösung aus II granuliert, getrocknet und gesiebt. Das Granulat wird mit den Substanzen unter III zur Tablettiermasse gemischt. Die Tablettierung erfolgt zu Tabletten von 7 mm Durchmesser und 140 mg Gewicht.

**Patentansprüche**

1. Aminopropanol-Derivate der allgemeinen Formel (I)

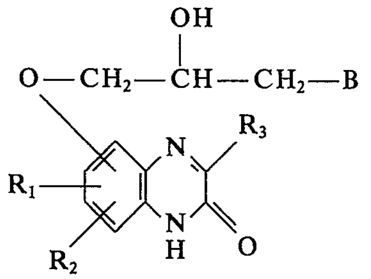

(I)

in der

$R_1$ und $R_2$ die gleich oder verschieden sein können, jeweils ein Wasserstoffatom oder eine $C_1-C_5$-Alkylgruppe oder gemeinsam einen $C_2-C_4$-Alkylenrest,

$R_3$ Wasserstoff oder einen $C_1-C_5$-Alkylrest, der gegebenenfalls durch Hydroxyl, Halogen, Phenyl oder $C_1-C_5$-Alkylthio substituiert ist, und

B einen $C_2-C_4$-Alkylaminorest, dessen Alkylteil gegebenenfalls einen Phenyl- oder Phenoxyrest trägt, der ein- oder mehrfach durch Halogen, Hydroxyl, $C_1-C_4$-Alkyl, $C_2-C_6$-Alkanoyl, Methylthio, $C_2-C_6$-Alkanoylamido, Aminocarbonyl, $C_1-C_4$-Alkoxy, Allyloxy, Phenoxy oder Trifluormethyl substituiert ist, oder einen Phenyloxymethylpiperidin-, Pyridyloxymethylpiperidin- oder Benzimidazolinonyloxymethylpiperidinrest, wobei der Phenylrest gegebenenfalls ein- oder mehrfach durch Halogen, Amino, Hydroxy, Carboxamido, eine $C_1-C_4$-Alkyl-, $C_2-C_6$-Alkanoyl- oder $C_1-C_4$-Alkoxygruppe substituiert ist,

bedeuten, sowie deren pharmakologisch unbedenkliche Salze.

2. Verfahren zur Herstellung von Aminopropanol-Derivaten der allgemeinen Formel (I)

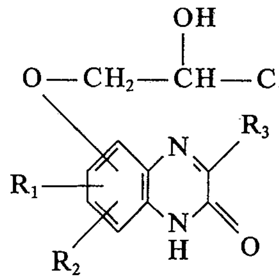

(I)

in der

| | |
|---|---|
| $R_1$ und $R_2$ | die gleich oder verschieden sein können, jeweils ein Wasserstoffatom oder eine $C_1-C_5$-Alkylgruppe oder gemeinsam einen $C_2-C_4$-Alkylenrest, |
| $R_3$ | Wasserstoff oder einen $C_1-C_5$-Alkylrest, der gegebenenfalls durch Hydroxyl, Halogen, Phenyl oder $C_1-C_5$-Alkylthio substituiert ist, und |
| B | einen $C_2-C_4$-Alkylaminorest, dessen Alkylteil gegebenenfalls einen Phenyl- oder Phenoxyrest trägt, der ein- oder mehrfach durch Halogen, Hydroxyl, $C_1-C_4$-Alkyl, $C_2-C_6$-Alkanoyl, Methylthio, $C_2-C_6$-Alkanoylamido, Aminocarbonyl, $C_1-C_4$-Alkoxy, Allyloxy, Phenoxy oder Trifluormethyl substituiert ist, oder einen Phenyloxymethylpiperidin-, Pyridyloxymethylpiperidin- oder Benzimidazolinonyloxymethylpiperidinrest, wobei der Phenylrest gegebenenfalls ein- oder mehrfach durch Halogen, Amino, Hydroxy, Carboxamido, eine $C_1-C_4$-Alkyl-, $C_2-C_6$-Alkanoyl- oder $C_1-C_4$-Alkoxygruppe substituiert ist, |

bedeuten, sowie deren pharmakologisch unbedenkliche Salze, dadurch gekennzeichnet, daß man in an sich bekannter Weise

a) eine Verbindung der allgemeinen Formel (II)

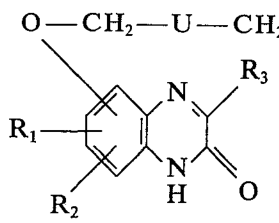

(II)

mit einer Verbindung der allgemeinen Formel (III)

$$H-B \qquad \text{(III)}$$

umsetzt, in denen

| | |
|---|---|
| $R_1$, $R_2$, $R_3$ und B | die oben angegebene Bedeutung haben, |
| V | einen nucleophil substituierbaren Rest und |
| U | die Gruppe $>C=O$ oder $>CH-OH$ darstellt oder |
| U | zusammen mit V eine Einfachbindung bildet, und falls U die Gruppe $>C=O$ bedeutet, anschließend reduziert, oder |

b) eine Verbindung der allgemeinen Formel (IV)

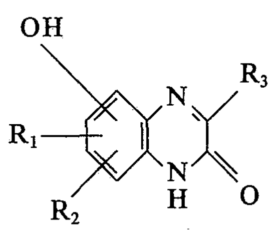

(IV)

mit einer Verbindung der allgemeinen Formel (V)

$$V-CH_2-U-CH_2-B \qquad \text{(V)}$$

umsetzt, in denen

$R_1$, $R_2$, $R_3$, B, U und V die oben angegebene Bedeutung haben, und falls U die Gruppe $>C=O$ bedeutet, anschließend reduziert, oder

c) für den Fall, daß $R_3 = CH_3$ vorstellt, eine Verbindung der allgemeinen Formel (VI)

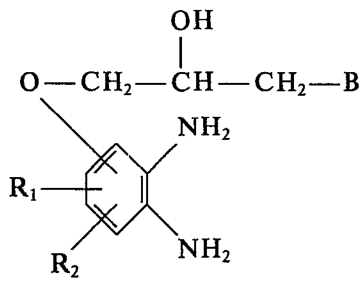

(VI)

in der

$R_1$, $R_2$ und B die angegebene Bedeutung haben, mit Acetylendicarbonsäure umsetzt, oder

d) eine Verbindung der allgemeinen Formel (VI) mit einer Verbindung der allgemeinen Formel (VII)

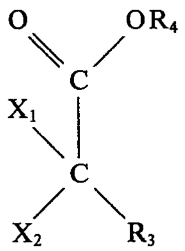

(VII)

in der

$R_3$ die oben angegebene Bedeutung hat,
$R_4$ Wasserstoff oder $C_1-C_4$-Alkyl,
$X_1$ Halogen und
$X_2$ Wasserstoff oder $X_1$ und $X_2$ zusammen ein Sauerstoffatom bedeuten,

umsetzt, und falls $X_2$ Wasserstoff darstellt, oxidiert, oder

e) eine Verbindung der allgemeinen Formel (VIII)

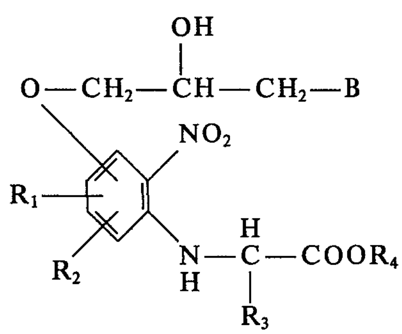

(VIII)

in der

$R_1$, $R_2$, $R_3$, $R_4$ und B die oben angegebene Bedeutung haben, reduziert, cyclisiert und oxidiert, oder

f) eine Verbindung der allgemeinen Formel (IX)

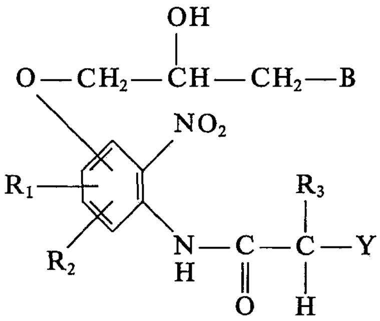

(IX)

in der

| | |
|---|---|
| $R_1$, $R_2$, $R_3$ und B | die oben angegebene Bedeutung haben und |
| Y | für eine nucleophil substituierbare Gruppe steht, reduziert, cyclisiert und oxidiert, |

und anschließend einen Substituenten im Rest B gewünschtenfalls in einen anderen Substituenten umwandelt und gegebenenfalls die erhaltenen Verbindungen der allgemeinen Formel (I) in ihre pharmakologisch verträglichen Salze überführt.

3. Arzneimittel, enthaltend einen Wirkstoff gemäß Anspruch 1 sowie an sich bekännte pharmakologisch verträgliche Träger- und Hilfsstoffe.

4. Verbindungen gemäß Anspruch 1 zur Verwendung bei der Bekämpfung von Herz- und Kreislauferkrankungen.

**Claims**

1. Aminopropanol derivatives of the general formula (I)

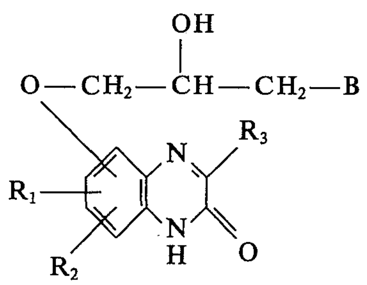

(I)

in which $R_1$ and $R_2$, which can be the same or different, each signify a hydrogen atom or a $C_1-C_5$ alkyl group or together a $C_2-C_4$ alkylene radical, $R_3$ a hydrogen atom or a $C_1-C_5$ alkyl radical, which is optionally substituted by hydroxyl, halogen, phenyl or $C_1-C_5$ alkylthio and B a $C_2-C_4$ alkylamino radical, the alkyl part of which optionally carries a phenyl or phenoxy radical, which is substituted one or more times by halogen, hydroxyl, $C_1-C_4$ alkyl, $C_2-C_6$ alkanoyl, methylthio, $C_2-C_6$ alkanoylamido, aminocarbonyl, $C_1-C_4$ alkoxy, allyloxy, phenoxy or trifluoromethyl, or is a phenoxy-methylpiperidine, pyridyloxymethylpiperidine or benzimidazolinonyloxymethylpiperidine radical, whereby the phenyl radical is optionally substituted one or more times by halogen, amino, hydroxyl, carboxamido, a $C_1-C_4$ alkyl, $C_2-C_6$ alkanoyl or $C_1-C_4$ alkoxy group; as well as their pharmacologically acceptable salts.

2. Process for the preparation of aminopropanol derivatives of the general formula (I)

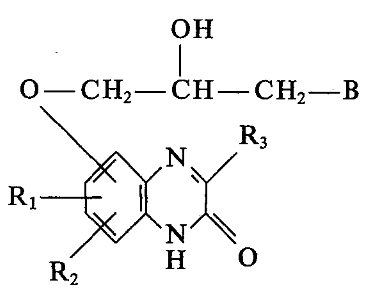

(I)

in which $R_1$ and $R_2$, which can be the same or different, each signify a hydrogen atom or a $C_1-C_5$ alkyl

group or together a $C_2-C_4$ alkylene radical, $R_3$ is a hydrogen atom or a $C_1-C_5$ alkyl radical, which is optionally substituted by hydroxyl, halogen, phenyl or $C_1-C_5$ alkylthio and B a $C_2-C_4$ alkylamino radical, the alkyl part of which optionally carries a phenyl or phenoxy radical, which is substituted one or more times by halogen, hydroxyl, $C_1-C_4$ alkyl, $C_2-C_6$ alkanoyl, methylthio, $C_2-C_6$ alkanoylamido, aminocarbonyl, $C_1-C_4$ alkoxy, allyloxy, phenoxy or trifluoromethyl; or is a phenoxymethylpiperidine, pyridyloxymethylpiperidine or benzimidazolinonyloxymethylpiperidine radical, whereby the phenyl radical is optionally substituted one or more times by halogen, amino, hydroxyl, carboxamido, a $C_1-C_4$ alkyl, $C_2-C_6$ alkanoyl or $C_1-C_4$ alkoxy group, as well as of their pharmacologically acceptable salts, characterised in that, in per se known manner, one

a) reacts a compound of the general formula (II)

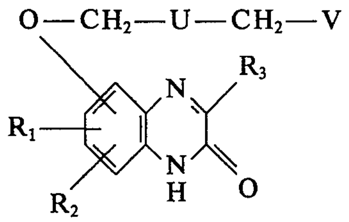

(II)

with a compound of the general formula (III)

$$H-B \qquad \text{(III)}$$

in which $R_1$, $R_2$, $R_3$ and B have the above-given meaning, V represents a nucleophilically substitutable residue and U the group $>C=O$ or $>CH-OH$ or U, together with V, forms a single bond, and, when U signifies the group $>C=O$, subsequently reduces; or

b) reacts a compound ot the general formula (IV)

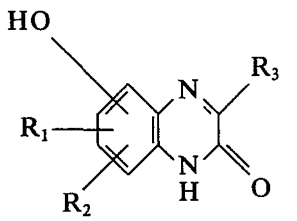

(IV)

with a compound of the general formula (V)

$$V-CH_2-U-CH_2-B \qquad \text{(V)}$$

in which $R_1$, $R_2$, $R_3$, B, U and V have the above-given meaning, and when U signifies the group $>C=O$, subsequently reduces; or

c) for the case in which $R_3=CH_3$, reacts a compound of the general formula (VI)

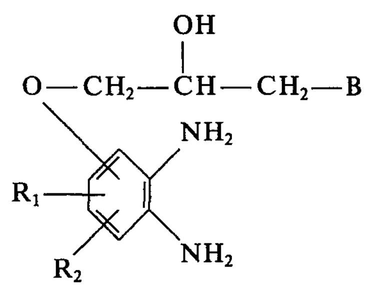

(VI)

in which $R_1$, $R_2$ and B have the given meaning, with acetylenedicarboxylic acid; or

d) reacts a compound of the general formula (VI) with a compund of the general formula (VII)

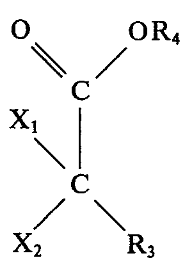

(VII)

in which $R_3$ has the above-given meaning, $R_4$ signifies hydrogen or $C_1-C_4$ alkyl, $X_1$ halogen and $X_2$ hydrogen or $X_1$ and $X_2$ together represent an oxygen atom, and, when $X_2$ represents hydrogen, oxidises, or

e) reduces, cyclises and oxidises a compound of the general formula (VIII)

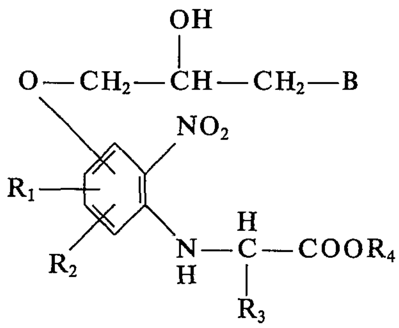

(VIII)

in which $R_1$, $R_2$, $R_3$, $R_4$ and B have the above-given meaning, or

f) reduces, cyclises and oxidises a compound of the general formula (IX)

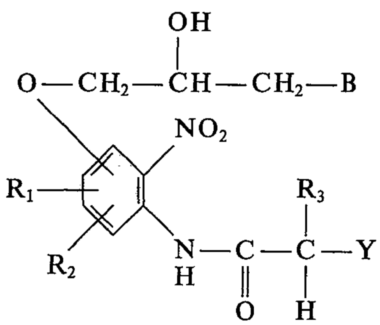

(IX)

in which $R_1$, $R_2$, $R_3$ and B have the above-given meaning and Y stands for a nucleophilically substitutable group, and subsequently, if desired, converts a substituent in the residue B into another substituent

and possibly converts the compounds obtained of general formula (I) into their pharmacologically acceptable salts.

3. Medicaments, containing an active material according to claim 1, as well as per se known pharmaceutically acceptable carrier and adjuvant materials.

4. Compounds according to claim 1 for use in the combating of heart and circulatory diseases.

**Revendications**

1. Dérivés d'aminopropanol de formule générale I

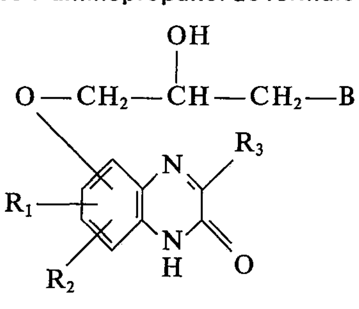

(I)

dans laquelle

$R_1$ et $R_2$ pouvant être identiques ou différents, sont chacun un atome d'hydrogène ou un groupe alkyle $C_1-C_5$ ou représentent ensemble un reste alkylène $C_2-C_4$,

$R_3$ est de l'hydrogène ou un reste alkyle $C_1-C_5$, éventuellement substitué par de l'hydroxyle, de l'halogène, du phényle ou de l'alkylthio $C_1-C_5$, et

B est un reste alkylamino $C_2-C_4$, dont la partie alkyle porte éventuellement un reste phényle ou phénoxy, substitué une ou plusieurs fois par de l'halogène, de l'hydroxyle, de

l'alkyle $C_1-C_4$, de l'alcanoyle $C_2-C_6$, du méthylthio, de l'alcanoylamido $C_2-C_6$, de l'aminocarbonyle, de l'alcoxy $C_1-C_4$, de l'allyloxy, du phénoxy ou du trifluorométhyle, ou est un reste phényloxyméthylpipéridine, pyridyloxyméthylpipéridine ou benzimidazolinonyloxyméthylpipéridine,
le reste phényle étant éventuellement substitué une ou plusieurs fois par de l'halogène, de l'amino, de l'hydroxyle, du carboxamido, un groupe alkyle $C_1-C_4$, alcanoyle $C_2-C_6$ ou alcoxy $C_1-C_4$,

ainsi que leurs sels pharmacologiquement irréprochables.

2. Procédé de préparation de dérivés d'aminopropanol de formule générale I

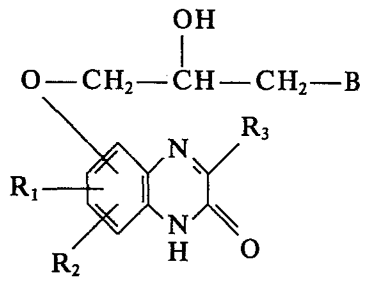

(I)

dans laquelle

| | |
|---|---|
| $R_1$ et $R_2$ | pouvant être identiques ou différents, sont chacun un atome d'hydrogène ou un groupe alkyle $C_1-C_6$ ou représentent ensemble un reste alkylène $C_2-C_4$, |
| $R_3$ | est de l'hydrogène ou un reste alkyle $C_1-C_5$ éventuellement substitué par de l'hydroxyle, de l'halogène, du phényle ou de l'alkylthio $C_1-C_5$, et |
| B | est un reste alkylamino $C_2-C_4$, dont la partie alkyle porte éventuellement un reste phényle ou phénoxy, substitué une ou plusieurs fois par de l'halogène, de l'hydroxyle, de l'alkyle $C_1-C_4$, de l'alcanoyle $C_2-C_6$, du méthylthio, de l'alcanoylamido $C_2-C_6$, de l'aminocarbonyle, de l'alcoxy $C_1-C_4$, de l'allyloxy, du phénoxy ou du trifluorométhyle, ou est un reste phényloxyméthylpipéridine, pyridyloxyméthylpipéridine ou benzimidazolinonyloxyméthylpipéridine, le reste phényle étant éventuellement substitué une ou plusieurs fois par de l'halogène, de l'amino, de l'hydroxyle, du carboxamido, un groupe alkyle $C_1-C_4$, un groupe alcanoyle $C_2-C_6$ ou alcoxy $C_1-C_4$, |

ainsi que leurs sels pharmacologiquement irréprochables, caractérisé en ce que de façon en soi connue:

a) on fait réagir un composé de formule générale II

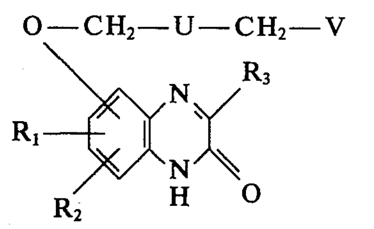

(II)

avec un composé de formule générale III

H−B (III)

dans lesquelles:

| | |
|---|---|
| $R_1$, $R_2$, $R_3$ et B | ont la signification donnée ci-dessus, |
| V | est un reste acceptant une substitution nucléophile et |
| U | est le groupe $>C=O$ ou $>CH-OH$ ou bien |
| U | forme avec V une liaison simple, et dans le cas où U est le groupe $>C=O$ on fait suivre une réduction, ou |

b) on fait réagir un composé de formule générale IV

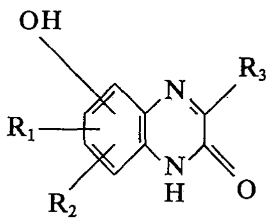

(IV)

avec un composé de formule générale V

$$V-CH_2-U-CH_2-B \qquad (V)$$

dans lesquelles:

$R_1$, $R_2$, $R_3$, B, U et V ont la signification indiquée ci-dessus, et, dans le cas où U est le groupe $>C=O$ ont fait suivre une réduction, ou

c) dans le cas où $R_3$ représente $CH_3$, on fait réagir un composé de formule générale VI

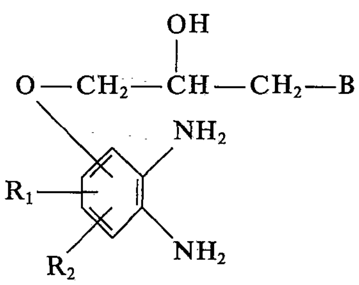

(VI)

dans laquelle:

$R_1$, $R_2$ et B ont la signification indiquée, avec l'acide acétylènedicarboxylique, ou

d) on fait réagir un composé de formule générale (VI) avec un composé de formule générale (VII)

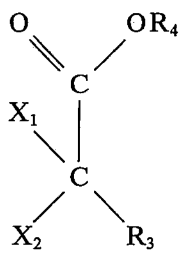

(VII)

dans laquelle:

$R_3$ a la signification donnée ci-dessus,
$R_4$ est de l'hydrogène ou de l'alkyle $C_1-C_4$,
$X_1$ est de l'halogène et
$X_2$ est de l'hydrogène ou bien $X_1$ et $X_2$ représentent ensemble un atome d'oxygène, et dans le cas où $X_2$ est de l'hydrogène, on effectue une oxydation, ou,

e) on soumet à une réduction, une cyclisation ou une oxydation un composé de formule générale VIII

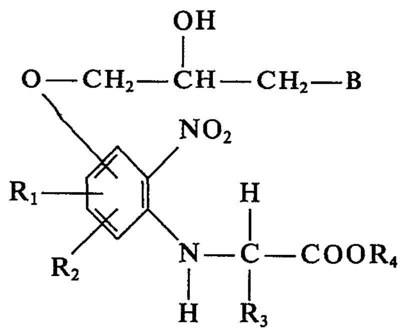

(VIII)

dans laquelle

$R_1$, $R_2$, $R_3$, $R_4$ et B ont la signification donnée ci-dessus, ou

f) ou soumet à une réduction, une cyclisation ou une oxydation, un composé de formule générale (IX)

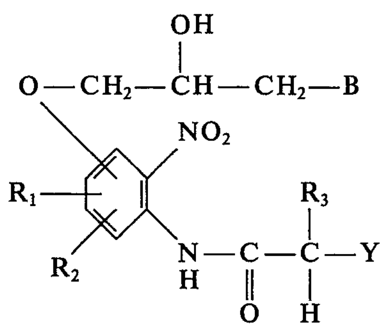

(IX)

dans laquelle:

$R_1$, $R_2$, $R_3$ et B ont la signification donnée ci-dessus et
Y représente un groupe acceptant une substitution nucléophile

et ensuite on transforme éventuellement un substituant du reste B en un autre substituant, et on transforme éventuellement les composés obtenus de formule générale (I) en leurs sels pharmacologiquement tolérables.

3. Médicaments, comprenant une substance active selon la revendication 1ainsi que des substances de support et auxiliaires en soi connues et pharmacologiquement tolérables.

4. Composés selon la revendication 1 pour la mise en œuvre dans les traitements des maladies du cœur et de la circulation.